(19) **Europäisches Patentamt / European Patent Office / Office européen des brevets**

(11) **EP 4 769 420 A1**

(12) **EUROPEAN PATENT APPLICATION**

(43) Date of publication:
01.07.2026 Bulletin 2026/27

(21) Application number: 25224732.5

(22) Date of filing: 18.12.2025

(51) International Patent Classification (IPC):
**G16H 10/20** (2018.01)    **G16H 10/40** (2018.01)

(52) Cooperative Patent Classification (CPC):
**G16H 10/20; G16H 10/40**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: 30.12.2024  CN 202411989436
19.05.2025  CN 202510641102

(71) Applicant: **Shenzhen Mindray Bio-Medical Electronics Co., Ltd.**
**Shenzhen, Guangdong 518057 (CN)**

(72) Inventors:
• **ZHENG, Wenbo**
**Shenzhen, 518057 (CN)**
• **MEI, Shaojie**
**Shenzhen, 518057 (CN)**
• **YE, Bo**
**Shenzhen, 518057 (CN)**

(74) Representative: **KIPA AB**
**Drottninggatan 11**
**252 21 Helsingborg (SE)**

(54) **METHOD AND APPARATUS FOR GENERATING OR USING REVIEW MODEL, AND STORAGE MEDIUM**

(57) The disclosure relates to a method for generating a review model for reviewing test data, including: obtaining test data and clinical information of a plurality of samples, wherein the test data is unlabeled and includes a test result for at least one test parameter, and the clinical information at least includes at least one of department and diagnosis result; and for each test parameter, constructing a review model corresponding to said test parameter by using a machine learning algorithm based on the test result corresponding to said test parameter in test data of each sample and the clinical information of said sample. The disclosure also relates to an apparatus for generating a review model for reviewing test data, a method and an apparatus for reviewing test data, and a computer-readable storage medium. The embodiments of the disclosure construct the review model in a simple way based on an unsupervised learning algorithm, which makes it possible to assist a user in quickly and accurately reviewing test data, thereby improving the review efficiency.

**100**

S110 — obtaining test data and clinical information of a plurality of samples

↓

S120 — for each test parameter, constructing a review model corresponding to said test parameter by using a machine learning algorithm based on the test result corresponding to said test parameter in test data of each sample and clinical information of said sample

FIG. 1

EP 4 769 420 A1

**Description**

TECHNICAL FIELD

The disclosure relates to the field of medical technology, and in particular, to a method for generating a review model for reviewing test data, a method for reviewing test data, an apparatus for generating a review model for reviewing test data, an apparatus for reviewing test data, and a computer-readable storage medium.

BACKGROUND

A clinical laboratory report is data information obtained through instrument testing and requires a review. Currently in clinical practice, the review is primarily conducted manually or conducted automatically based on simple rules and logic. Manual reviews require a high level of expertise from clinicians, exhibit high subjectivity, and lack uniform standards, and thus cannot guarantee review quality, and fail to meet the rapidly growing demand for large-scale clinical reviews.

Compared to manual reviews, rule-based automated reviews can effectively improve review efficiency, but only achieve a 50% to 70% review pass rate, requiring frequent human intervention. With the increasing demand from clinicians and patients for shorter test turnaround time (TAT) and report harmonization, improving the pass rate of automated report reviews is an urgent clinical need.

SUMMARY

Based on this, an object of the disclosure is to provide a technical solution for establishing an intelligent review model for reviewing clinical laboratory results based on an unsupervised learning algorithm.

According to a first aspect of the disclosure, a method for generating a review model for reviewing test data is provided, including:

obtaining test data and clinical information of a plurality of samples, wherein the test data is unlabeled, the test data includes a test result for at least one test parameter, and the clinical information at least includes at least one of department and diagnosis result; and

for each test parameter of the at least one test parameter, constructing a review model corresponding to said test parameter by using a machine learning algorithm based on the test result corresponding to said test parameter in the test data of each sample of the plurality of samples and the clinical information of said sample.

According to a second aspect of the disclosure, a method for reviewing test data is provided, including:

obtaining a test result for a current test parameter of a current sample and clinical information of the current sample;

determining a current review model from a plurality of review models based on the current test parameter and the clinical information, wherein the plurality of review models are constructed according to the method according to the first aspect of the disclosure;

performing a review on the test result for the current test parameter based on the current review model; and

outputting, based on a result of the review, the test result for the current test parameter or an alarm prompt and/or a manual review prompt.

According to a third aspect of the disclosure, an apparatus for generating a review model for reviewing test data is provided, including:

a non-transitory computer-readable storage medium storing computer-readable instructions; and

one or more processors configured to execute the computer-readable instructions to implement the method of the first aspect of the disclosure.

According to a fourth aspect of the disclosure, an apparatus for reviewing test data is provided, including:

a non-transitory computer-readable storage medium storing computer-readable instructions; and

one or more processors configured to execute the computer-readable instructions to implement the method of the second aspect of the disclosure.

According to a fifth aspect of the disclosure, a computer-readable storage medium is provided, which has a computer program stored thereon, wherein the computer program, when executed by a processor, implements the method of the first aspect of the disclosure or the second aspect of the disclosure.

In the technical solutions proposed in various aspects of the disclosure, using an unsupervised learning algorithm enables the review model for automated review of test data to be constructed in a simple way and with low resource consumption. Furthermore, considering at least one of department and diagnosis result of samples during the construction of the review model significantly improves the review pass rate and reduces human intervention, thereby reducing the review burden on a clinician.

BRIEF DESCRIPTION OF THE DRAWINGS

FIG. 1 is a schematic flowchart of a method for generating a review model for reviewing test data according to some embodiments of the disclosure.
FIG. 2 is a scoring curve for evaluating clustering effect when performing K-means clustering on departments for UA parameter according to some embodiments of the disclosure.
FIG. 3 is an effect diagram of performing K-means clustering on diagnosis results for UREA-Cr parameter combination according to some embodiments of the disclosure.
FIG. 4 is a correlation curve between UA parameter and $CO_2$ parameter according to some embodiments of the disclosure.
FIG. 5 is a correlation curve between UREA parameter and Cr parameter according to some embodiments of the disclosure.
FIG. 6 is a correlation curve between UREA parameter and CYSC parameter according to some embodiments of the disclosure.
FIG. 7 is a correlation curve between Cr parameter and CYSC parameter according to some embodiments of the disclosure.
FIG. 8 is a $CO_2$ review model constructed for first clinical information according to some embodiments of the disclosure.
FIG. 9 is a $CO_2$ review model constructed for second clinical information according to some embodiments of the disclosure.
FIG. 10 is a UREA-Cr review model constructed for third clinical information according to some embodiments of the disclosure.
FIG. 11 is a UREA-Cr review model constructed for fourth clinical information according to some embodiments of the disclosure.
FIG. 12 is a schematic flowchart of a method for reviewing test data according to some embodiments of the disclosure.
FIG. 13 is a schematic diagram of a sample review according to some embodiments of the disclosure.

DESCRIPTION OF THE PREFERRED EMBODIMENTS

The technical solutions of the embodiments of the disclosure will be described below clearly and comprehensively with reference to accompanying drawings of the embodiments of the disclosure. Apparently, the embodiments described are merely some of, rather than all of, the embodiments of the disclosure. Based on the embodiments of the disclosure, all the other embodiments which would have been obtained by those of ordinary skill in the art without any creative efforts shall fall within the scope of protection of the disclosure.

It should be noted that the term "first/second/third" in the embodiments of the disclosure is only used to distinguish similar objects, and does not represent specific order for the objects. It may be understood that "first/second/third" may be interchanged for specific order or chronological order when allowed.

In the related art, a rule-based automated review only considers device testing-related information, including a distribution range of item results, consistency with historical results, critical values, correlation among different items, and presence of an abnormal alarm.

However, in clinical practice, laboratory results of some abnormal samples are strongly correlated with patient clinical information. For example, renal function test results of a patient with end-stage uremia are likely to be extremely abnormal. Therefore, incorporation of clinical information of patients in reviewing diagnosis results of the patients is one of key paths to improve the review pass rate.

In the related art, machine learning-based artificial intelligence review methods are proposed, wherein an intelligent review model is constructed through supervised training of a classifier with a large amount of data. However, in such a method, a clinician is required to complete annotation of a large amount of test data. However, the annotation of clinical test data is a complex and demanding task, which not only requires a high level of professional expertise from annotators and depends on the subjective cognition of the annotators, but also is time-consuming and labor-intensive, leading to a waste of already limited medical resources.

Based on this, embodiments of the disclosure propose constructing a review model for automated review of clinical laboratory results using an unsupervised learning algorithm based on test data and clinical information of historical samples, so that an intelligent review model can be constructed in a simple and low-resource-consumption manner and under consideration of clinical information.

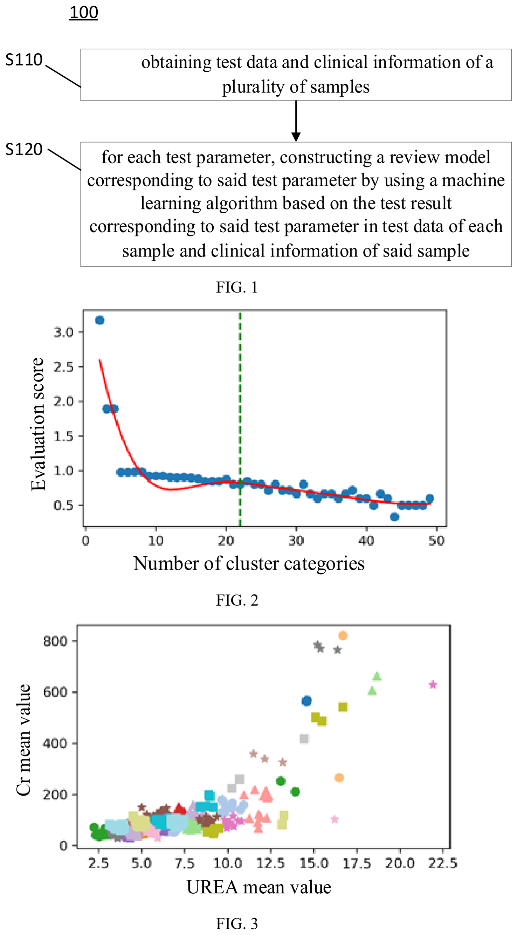

As shown in FIG. 1, an embodiment of the disclosure first provides a method 100 for generating a review model for reviewing test data.

Step S110: obtaining test data and clinical information of a plurality of samples (i.e., historical samples). Here, the test data of these samples is unlabeled, i.e., unannotated or unmarked. Moreover, the test data includes a test result for at least one test parameter, and the clinical information at least includes at least one of department and diagnosis result, for example, the clinical information at least includes department and diagnosis result.

In some embodiments, the test data being unlabeled may indicate that the test data is not annotated with at least one of whether retesting is performed, whether an abnormality is present, or whether review approval is granted. In particular, test data being unlabeled indicates that the test data is not annotated with whether retesting is performed, is not annotated with whether an abnormality is present, and is not annotated with whether a review approval is granted.

In some embodiments, the clinical information may further include gender and age. Preferably, the clinical information includes department, diagnosis result, gender, and age.

In a specific example, test data and clinical information of n samples, as shown in Tables 1 and 2, may be obtained, wherein the displayed test data includes test results of six test parameters: uric acid (UA), urea (UREA), cystatin (CYSC), creatinine (Cr), carbon dioxide combining power ($CO_2$), and Glucose (GLU), and the clinical information includes department, diagnosis result, gender, and age. Some specific examples of the disclosure will be described below with Tables 1 and 2 as examples, but the embodiments of the disclosure are not limited thereto.

Table 1. Test Data of a Plurality of Samples

| Sample | UA | UREA | CYSC | Cr | CO2 | GLU |
|--------|-----|------|------|-----|-----|-----|
| S1 | A1 | B1 | C1 | D1 | E1 | F1 |
| S2 | A2 | B2 | C2 | D2 | E2 | F2 |
| S3 | A3 | B3 | C3 | D3 | E3 | F3 |
| S4 | A4 | B4 | C4 | D4 | E4 | F4 |
| S5 | A5 | B5 | C5 | D5 | E5 | F5 |
| ... | ... | ... | ... | ... | ... | ... |
| Sn | An | Bn | Cn | Dn | En | Fn |

Table 2. Clinical Information of the Samples in Table 1

| Sample | Department | Diagnosis Result | Gender | Age |
|--------|-----------|------------------|--------|-----|
| S1 | Orthopedics and Joint Surgery, Spine Surgery | Clavicle Fracture, Cervical Spondylosis | Male | 24 |
| S2 | Comprehensive Ward, Hematology | Chemotherapy for Malignant Lymphoma | Female | 42 |
| S3 | Cardiovascular Medicine | Arrhythmia, Hypertension | Male | 69 |
| S4 | Infectious Diseases | Chronic Hepatitis B | Male | 53 |
| S5 | Nephrology | Renal Insufficiency, Chronic Nephritis Syndrome | Male | 43 |
| ... | ... | ... | ... | ... |
| Sn | Respiratory Medicine, Neurology | Pulmonary Infection, Limb Numbness | Female | 37 |

Step S120: for each test parameter of the at least one test parameter, constructing a review model corresponding to said test parameter by using a machine learning algorithm based on the test result corresponding to said test parameter in the test data of each sample of the plurality of samples and the clinical information of said sample.

For example, taking the data in Table 1 as an example, step S120 may be specifically implemented as follows: for the test parameter UA, constructing a review model corresponding to UA by using a machine learning algorithm based on the UA test result for each sample of the samples S1 to Sn and the clinical information of said sample; for the test parameter UREA, constructing a review model corresponding to UREA by using a machine learning algorithm based on the UREA test result for each sample of the samples S1 to Sn and the clinical information of said sample; for the test parameter CYSC, constructing a review model corresponding to CYSC by using a machine learning algorithm based on the CYSC test result for each sample of the samples S1 to Sn and the clinical information of said sample; and for the test parameter Cr, constructing a review model corresponding to Cr by using a machine learning algorithm based on the Cr test result for each sample of the samples S1 to Sn and the clinical information of said sample, and so on.

Here, since the test data is unlabeled, that is, the review model is constructed based on an unsupervised learning algorithm, there is no need for manual labeling of the historical test data used for model construction, which reduces the cost of model construction. In addition, clinical information of patients is considered during construction of the review model. Therefore, the review model constructed in this way allows for reduction in human intervention by a clinician in a process of reviewing test data and improves the review pass rate.

In some embodiments, in step S120, constructing a review model corresponding to said test parameter by using a machine learning algorithm based on the test result corresponding to said test parameter in the test data of each sample of the plurality of samples and the clinical information of said sample, may include:

performing clustering on the clinical information of the plurality of samples; and
constructing the review model corresponding to said test parameter based on the test result corresponding to said test parameter in the test data of each sample of the plurality of samples and the clustered clinical information of said sample.

In other words, the clinical information of the plurality of samples is divided into a plurality of categories in order to reduce the number of review models.

In some implementations, the clinical information may at least include department. Correspondingly, performing clustering on the clinical information of the plurality of samples includes performing clustering on the departments of the plurality of samples.

For example, k1 departments may be divided into m1 categories, wherein m1 is less than k1. Therefore, the department to which each sample belongs correspondingly belongs to at least one of these m1 categories.

In some other implementations, the clinical information may at least include department and diagnosis result. Correspondingly, performing clustering on the clinical information of the plurality of samples includes respectively performing clustering on the departments of the plurality of samples and the diagnosis results of the plurality of samples.

For example, k1 departments may be divided into m1 categories, and k2 diagnosis results may be divided into m2 categories, wherein m1 is less than k1 and m2 is less than k2. Therefore, the department to which each sample belongs correspondingly belongs to at least one of these m1 department categories, and the diagnosis result to which each sample belongs correspondingly belongs to at least one of these m2 diagnosis result categories.

In still some other implementations, the clinical information may include department, diagnosis result, gender, and age. Accordingly, performing clustering on the clinical information of the plurality of samples includes respectively performing clustering on the departments, the diagnosis results, the genders, and the ages of the plurality of samples.

For example, as shown in Table 3, the departments may be divided into m1 categories, the diagnosis results may be divided into m2 categories, the genders may be divided into two categories, male and female, and the ages may be divided into m3 categories according to age groups. Therefore, the department to which each sample belongs correspondingly belongs to at least one of the m1 department categories, the diagnosis result to which each sample belongs correspondingly belongs to at least one of the m2 diagnosis result categories, the gender to which each sample belongs is either male or female, and the age to which each sample belongs correspondingly belongs to one of the m3 categories.

Table 3. Clustering or Division Results of Clinical Information

| Clinical Information | Number of Categories |
| --- | --- |
| Department | m1 |
| Diagnosis Result | m2 |
| Gender | 2 |
| Age | m3 |

In some embodiments, in step S120, performing clustering on the clinical information of the plurality of samples may include: performing clustering on the clinical information of the plurality of samples based on a preset clustering rule, for example a manually set clustering rule.

In some implementations, the clustering rule may be preset based on clinical experience. For example, the departments may be divided into m1 categories, such as 20 categories, based on clinical experience, and the diagnosis results may be divided into m1 categories, such as 40 categories, based on clinical experience.

In some other implementations, the clustering rule may be preset based on general common knowledge. For example, based on common knowledge, the genders may be divided into two categories: male and female.

In still some other implementations, the clustering rule may be preset based on national standards or international standards. For example, based on the national standards, the ages may be divided into infants (birth to 1 year old), toddlers (1 to 4 years old), children (5 to 11 years old), adolescents (12 to 18 years old), young adults (19 to 35 years old), middle-aged adult (36 to 59 years old), and elderly (60 years old and over).

It should be understood here that the clustering rule may be user-defined.

In some other embodiments, in step S120, performing clustering on the clinical information of the plurality of samples may include: performing clustering on the clinical information of the plurality of samples based on the test result corresponding to said test parameter in the test data of each sample of the plurality of samples.

In other words, for each test parameter, a clustering algorithm is used separately to perform clustering on the clinical information based on the test result corresponding to the test parameter in the test data of each sample of the plurality of samples.

A specific example of performing clustering on the clinical information is described by taking a K-means clustering algorithm as an example. It should be understood that the embodiments of the disclosure are not limited to the K-means clustering algorithm, and other clustering algorithms may also be used, for example, mean drift clustering, density-based clustering (DBSCAN) methods, Gaussian mixture model clustering, and the like.

In an example, taking performing clustering on the departments for the uric acid (UA) parameter as an example, a score for evaluating the clustering effect is calculated using the following formula:

$$\text{score} = \frac{\Delta d_i}{d_{mean}} \times 100\%$$

wherein $d_{mean}$ denotes the mean value of all clusters, and $\Delta d_i$ denotes the distance from the mean value of the i-th cluster to the mean value of all clusters. As shown in FIG. 2, the distribution of the scores (blue dots) as the number of clusters increases is plotted, and the scores are fitted into a smooth red curve. The position where the curve no longer drops significantly (the number of clusters increases, and the decrease in scores for three consecutive scores is less than 5%) is taken as a target number of clusters, such as the position of the green line, at which N=23. After the number of clusters is determined, K-means clustering is performed on the departments for the UA parameter, dividing the departments into 23 categories, as shown in Table 4.

Table 4. Department Clustering Results for UA Parameter

| Category | Department Information |
|---|---|
| 1 | Pain Management and Treatment, Cardiac Surgery |
| 2 | Emergency Comprehensive Ward, Emergency Department |
| 3 | Laminar Flow Room, Interventional Ultrasound, Hepatobiliary and Pancreatic Surgery, Infectious Diseases Department, Nuclear Medicine, Respiratory Medicine, etc. |
| 4 | ICU, Isolation Ward |
| 5 | Baby-Friendly Area, Delivery Room |
| 6 | Rheumatology and Immunology, Physical Examination Department |
| ...... | |
| 23 | Specialty Clinic, General Outpatient Service |

In another example, clustering, for example, K-means clustering, may also be performed on the clinical information for a plurality of test parameters. Taking a combination of the urea (UREA) parameter and the creatinine (Cr) parameter as an example (the situation is similar for other combinations involving more test parameters), the number of clusters for the UREA-Cr parameter combination is determined in a manner similar to the K-means clustering of the departments for the uric acid (UA) parameter as described above, and then K-means clustering is performed on the diagnosis results, as shown in FIG. 3.

In some embodiments, in step S120, the review model corresponding to said test parameter is constructed based on a distribution of the test result corresponding to said test parameter in the test data of each sample of the plurality of samples across the clustered clinical information.

For example, taking Tables 1 and 4 as an example, for the uric acid (UA) parameter, a review model corresponding to the UA parameter is constructed based on a distribution of the UA test results A1 to An of the samples S1 to Sn across the clustered 23 departments.

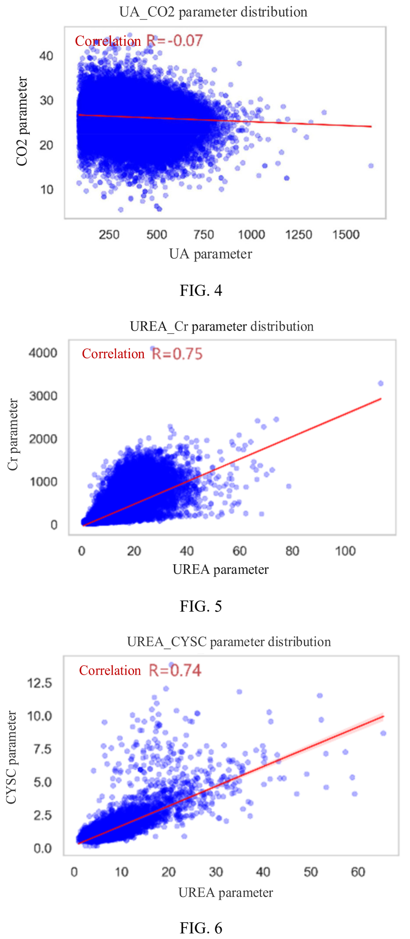

For another example, taking Table 1 and FIG. 4 as an example, for the combination of the urea (UREA) parameter and the creatinine (Cr) parameter, a review model corresponding to the UREA-Cr parameter combination is constructed based on a distribution of the UREA test results B1 to Bn and the creatinine (Cr) test results D1 to Dn of the samples S1 to Sn across the clustered diagnosis results.

In some embodiments, the at least one test parameter includes a plurality of test parameters. In step S120, prior to performing clustering on the clinical information of the plurality of samples, the method 100 further includes: grouping the plurality of test parameters to obtain a plurality of parameter groups, each parameter group consisting of a single test parameter of the plurality of test parameters or consisting of multiple test parameters of the plurality of test parameters. Accordingly, in step S120, constructing the review model corresponding to said test parameter based on the test result corresponding to said test parameter in the test data of each sample of the plurality of samples and the clustered clinical information of said sample, includes: constructing a review model corresponding to the parameter group containing said test parameter based on a distribution of the test result corresponding to each test parameter in the parameter group containing said test parameter in the test data of each sample of the plurality of samples across the clustered clinical information, as the review model corresponding to said test parameter.

In other words, constructing the review model for each parameter group here can simplify the review model. The review model constructed for each parameter group is configured to jointly review all test parameters contained in said parameter group.

Taking Table 1 as an example, the six test parameters UA, UREA, CYSC, Cr, CO2, and GLU may be combined into one parameter group, and then a review model may be constructed only for this parameter group, and this review model is used to jointly review these six test parameters.

Alternatively, the six test parameters UA, UREA, CYSC, Cr, CO2, and GLU may be divided into four parameter groups, wherein a first parameter group consists of UA, a second parameter group consists of UREA, CYSC, and Cr, a third parameter group consists of CO2, and a fourth parameter group consists of GLU. Then, a review model is constructed for each of the first parameter group, the second parameter group, the third parameter group, and the fourth parameter group, wherein the review model constructed for the first parameter group is used to individually review the UA parameter, the review model constructed for the second parameter group is used to jointly review the UREA, CYSC, and Cr parameters, the review model constructed for the third parameter group is used to individually review the CO2 parameter, and the review model constructed for the fourth parameter group is used to individually review the GLU parameter.

In some implementations, grouping the plurality of test parameters to obtain a plurality of parameter groups may include grouping the plurality of test parameters based on a preset grouping rule, for example, based on a manually set grouping rule.

Optionally, the grouping rule may be set based on clinical experience.

In some other implementations, grouping the plurality of test parameters to obtain a plurality of parameter groups may include grouping the plurality of test parameters based on a correlation between the test results for every two test parameters of the plurality of test parameters.

For example, taking the six test parameters in Table 1 as an example, the correlation between the UA test parameter and each of the other five test parameters of the n samples is calculated, wherein FIG. 4 shows the correlation between the UA test parameter and the CO2 test parameter. The UA test parameter has no correlation with any of the other five test parameters, so the UA test parameter is separately grouped into one parameter group (also known as a single-parameter group). Furthermore, through correlation analysis, the CO2 test parameter has no correlation with any of the other five test parameters, and the GLU test parameter has no correlation with any of the other five test parameters, so the CO2 test parameter and the GLU test parameter are also respectively grouped into one parameter group (also known as a single-parameter group).

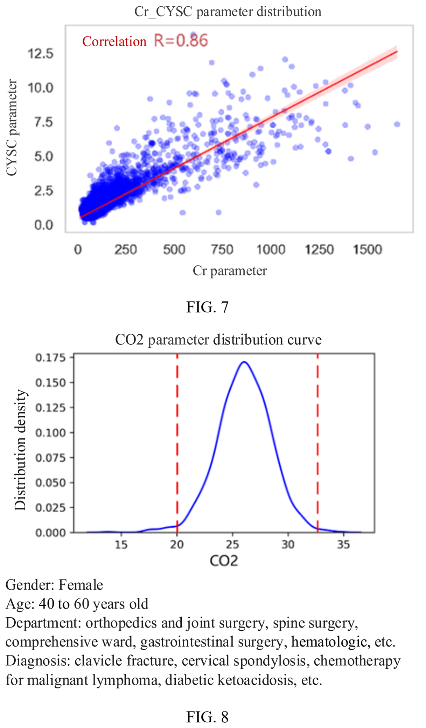

For another example, taking the three test parameters UREA, CYSC, and Cr in Table 1 as an example, the correlation between every two parameters among the UREA, CYSC, and Cr test parameters is calculated respectively. That is, the correlation between the UREA test parameter and the Cr test parameter of the n samples is calculated, as shown in FIG. 5; the correlation between the UREA test parameter and the CYSC test parameter of the n samples is calculated, as shown in FIG. 6; and the correlation between the Cr test parameter and the CYSC test parameter of the n samples is calculated, as shown in FIG. 7. As can be seen from FIG. 5 to FIG. 7, there is correlation between every two of the three parameters: the urea UREA, the cystatin CYSC, and the creatinine Cr. Therefore, these three parameters may be grouped into one parameter group (also known as a three-dimensional parameter group), or any two of these three parameters may be grouped into one parameter group (also known as a two-dimensional parameter group).

**In** some embodiments, in step S120, for each test parameter of the at least one test parameter, constructing a review model corresponding to said test parameter by using a machine learning algorithm based on the test result corresponding to said test parameter in the test data of each sample of the plurality of samples and the clinical information of said sample, may include:

performing clustering on the clinical information of the plurality of samples based on the test result corresponding to each test parameter in the parameter group containing said test parameter in the test data of each sample of the plurality of samples;

classifying the plurality of samples, and preferably classifying the plurality of samples based on the clustered clinical information, to obtain a plurality of sample groups; and

determining a review model corresponding to each sample group based on a distribution of the test result corresponding to each test parameter in the parameter group containing said test parameter in test data of each sample in said sample group across the clustered clinical information, as the review model corresponding to said test parameter.

For example, in step S120, after grouping the plurality of test parameters to obtain a plurality of parameter groups, for each parameter group, clustering is first performed on the clinical information of the plurality of samples based on the test result corresponding to each test parameter in said parameter group in the test data of each sample of the plurality of samples; then, the plurality of samples are classified based on the clustered clinical information to obtain a plurality of sample groups; and next, a review model corresponding to each sample group is determined based on a distribution of the test result corresponding to each test parameter in said parameter group in the test data of each sample in said sample group.

Now, a specific process for constructing a review model corresponding to a single-parameter group consisting of the UA parameter is described below by taking Tables 1, 2, and 3 as an example. Step 1: dividing the departments into m1 categories by means of K-means clustering, dividing the diagnosis results into m2 categories by means of K-means clustering, dividing the genders into two categories, male and female, and dividing the ages into m3 categories according to age groups, thereby obtaining a total of m1 × m2 × 2 × m3 categories of clinical information, with the division situation as shown in Table 3; step 2: classifying the n samples into m1 × m2 × 2× m3 sample groups based on the category to which each of department, diagnosis result, gender, and age of each sample belongs; and step 3: for each sample group, determining a review model corresponding to said sample group based on a distribution of the UA test results of each sample in said sample group. It should be understood here that, for the UA parameter, the number of review models is theoretically also m1 × m2 × 2 × m3, that is, one sample group corresponds to one review model.

It should be understood here that the above procedure is also applicable to a multi-dimensional parameter group consisting of multiple test parameters, for example, a two-dimensional parameter group consisting of two test parameters and a three-dimensional parameter group consisting of three test parameters. For example, the difference only lies in that, for each sample group, a review model corresponding to said sample group is determined based on a distribution of the test results for the multiple test parameters of each sample in said sample group.

It should be understood here that a one-dimensional review model is generated for a single-parameter group, and a multi-dimensional review model is generated for a multi-dimensional parameter group.

In some embodiments, for a multi-dimensional parameter group consisting of at least three test parameters, the corresponding multi-dimensional review model may be jointly represented by two-dimensional review models that are each constructed based on pairs of test parameters among the at least three test parameters. For example, for a three-dimensional parameter group consisting of three test parameters UREA, Cr, and CYSC, a three-dimensional review model corresponding to this three-dimensional parameter group may be jointly represented by a two-dimensional review model constructed based on UREA and Cr, a two-dimensional review model constructed based on UREA and CYSC, and a two-dimensional review model constructed based on Cr and CYSC.

In some embodiments, the parameter group containing said test parameter includes a parameter group consisting of a single test parameter. That is, for each of the plurality of parameter groups that consists of a single test parameter, determining a review model corresponding to each sample group based on a distribution of the test result corresponding to each test parameter in the parameter group containing said test parameter in test data of each sample in the sample group across the clustered clinical information, may include: determining a review threshold corresponding to each sample group based on a one-dimensional distribution of the test result corresponding to the single test parameter in the parameter group consisting of the single test parameter in the test data of each sample in the sample group across the clustered clinical information, to determine the review model corresponding to said sample group.

Optionally, the one-dimensional distribution may be a kernel density distribution or a Gaussian distribution. Further, review thresholds are determined based on a peak value of the kernel density distribution or the Gaussian distribution. The review model includes the kernel density distribution or the Gaussian distribution and the corresponding review thresholds.

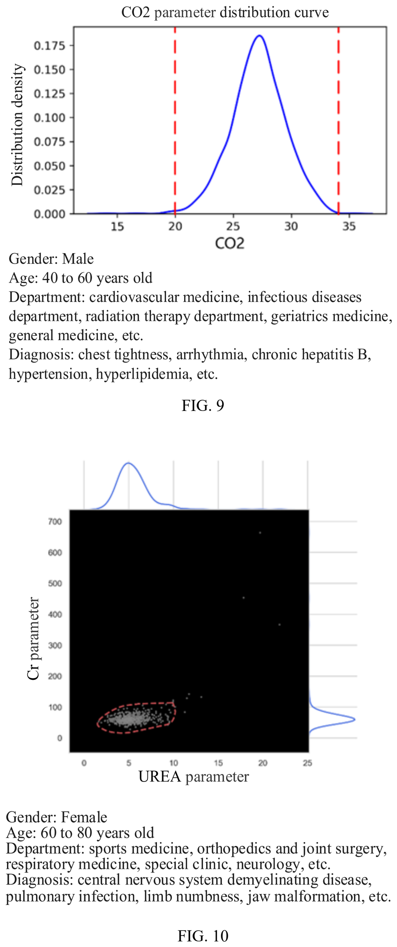

Taking the CO2 parameter in Table 1 as an example, CO2 review models corresponding to two categories of clinical information are constructed, as shown in FIG. 8 and FIG. 9. FIG. 8 shows a one-dimensional distribution of CO2 corresponding to a first category of clinical information, wherein first category of clinical information includes: gender being female; age being between 40 and 60 years old; departments including orthopedics and joint surgery, spine surgery, comprehensive ward, gastrointestinal surgery, hematology, etc.; and diagnosis results including clavicle fracture, cervical spondylosis, chemotherapy for malignant lymphoma, diabetic ketoacidosis, etc. FIG. 9 shows a one-dimensional distribution of CO2 corresponding to a second category of clinical information, wherein the second category of clinical information includes: gender being male; age being between 40 and 60 years old; departments including cardiovascular medicine, infectious diseases department, radiation therapy department, geriatrics medicine, general medicine, etc.; and diagnosis results including chest tightness, arrhythmia, chronic hepatitis B, hypertension, hyperlipidemia, etc. **In** the one-dimensional distribution curves shown in FIG. 8 and FIG. 9, the highest peak of the curve is first identified and a CO2 value corresponding to the highest peak is determined, and then starting from the highest peak towards both sides, thresholds on the left and right sides are identified, as indicated by red lines in the figures. The left and right thresholds are used as review thresholds for the review model. If a CO2 test result is within the review threshold range, it is indicated that the review passes; if the CO2 test result is outside the review threshold range, it is indicated that the review fails.

Alternatively or additionally, in some embodiments, in step S120, the parameter group containing said test parameter includes a parameter group consisting of multiple test parameters. That is, for each of the plurality of parameter groups that consists of multiple test parameter, determining a review model corresponding to each sample group based on a distribution of the test result corresponding to each test parameter in the parameter group containing said test parameter in test data of each sample in the sample group across the clustered clinical information, may include:
determining a review threshold corresponding to each sample group based on a multi-dimensional distribution of the test results corresponding to the multiple test parameters in the parameter group consisting of the multiple test parameters in the test data of each sample in the sample group across the clustered clinical information, to determine the review model corresponding to said sample group.

Optionally, the multi-dimensional distribution may be a kernel density distribution. Taking a two-dimensional parameter group as an example, a two-dimensional kernel density distribution may be generated based on test results of two test parameters, and review thresholds may be determined based on the position of the highest density. The review model may include the kernel density distribution and the corresponding review thresholds.

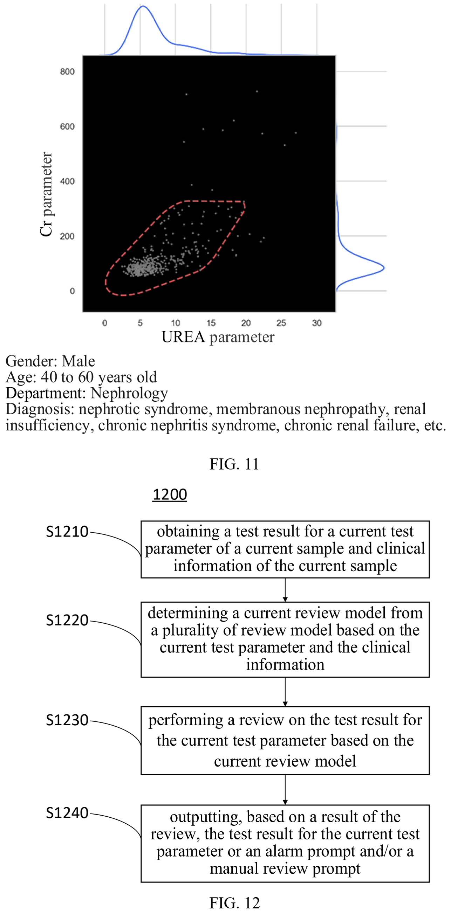

Taking a two-dimensional parameter group consisting of the UREA parameter and the Cr parameter as an example, UREA-Cr review models corresponding to two categories of clinical information are constructed, as shown in FIG. 10 and FIG. 11. FIG. 10 shows a UREA-Cr kernel density distribution corresponding to a third category of clinical information, wherein the third category of clinical information includes: gender being female; age being between 60 and 80 years old; departments including sports medicine, orthopedics and joint surgery, respiratory medicine, special clinic, neurology, etc.; and diagnosis results including central nervous system demyelinating disease, pulmonary infection, limb numbness, jaw malformation, etc. FIG. 11 shows a UREA-Cr kernel density distribution corresponding to a fourth category of clinical information, wherein the fourth category of clinical information includes: gender being male; age being between 40 to 60 years old; departments including nephrology; and diagnosis results including nephrotic syndrome, membranous nephropathy, renal insufficiency, chronic nephritis syndrome, chronic renal failure, etc. In the kernel density distributions shown in FIG. 10 and FIG. 11, the position of the highest density is first identified, and then contour lines are traversed outward from the position of the highest density until the ratio of the area of an inner contour line to the area of an outer contour line or the ratio of the numbers of internal points thereof is less than a threshold (e.g., 0.9). The contour line identified at this point (indicated by a red dash line) is determined as the desired review thresholds. If a UREA-Cr test result is within the determined contour line, it is indicated that the review passes; if the UREA-Cr test result is outside the determined contour line, it is indicated that the review fails.

Optionally, the multi-dimensional distribution may also be a multi-dimensional Gaussian distribution. Further, review thresholds are determined based on the multi-dimensional Gaussian distribution. The review model includes the multi-dimensional Gaussian distribution and corresponding review thresholds.

In some other embodiments, in step S120, the parameter group containing said test parameter includes a parameter group consisting of multiple test parameters. That is, for each of the plurality of parameter groups that consists of multiple test parameter, determining a review model corresponding to each sample group based on a distribution of the test result corresponding to each test parameter in the parameter group containing said test parameter in test data of each sample in the sample group across the clustered clinical information, may include: determining a review threshold corresponding to each sample group based on a one-dimensional distribution of the test results corresponding to the multiple test parameters in the parameter group consisting of the multiple test parameters in the test data of each sample in the sample group across the clustered clinical information, to determine the review model corresponding to said sample group.

In some embodiments, prior to constructing the review model, data preprocessing is performed on the test data and the clinical information of each sample of the plurality of samples, the data preprocessing including at least one of data cleaning, data deduplication, data imputation, and quantification of the clinical information, so as to construct the review model based on the preprocessed test data and the preprocessed clinical information.

Preferably, the data preprocessing includes data cleaning, data deduplication, data imputation, and quantification of the clinical information.

In some embodiments, the clinical information may be quantified as fixed integers.

In an example, age may be quantified as a fixed numerical value from 0 to 150. That is, if the age of a patient to whom a sample belongs is 30, the age is quantified to 30. In other examples, age may be quantified as a percentage. For example, if the age to which a sample belongs is 30, the age is quantified to 30/100.

In an example, gender may be quantified into a first positive integer or a second positive integer, wherein the first positive integer represents male, and the second positive integer represents female. For example, the first positive integer is 1, and the second positive integer is 2, but the embodiments of the disclosure are not limited thereto.

Alternatively or additionally, the clinical information may be quantified using a lookup table method.

In an example, various different diagnosis results may be assigned corresponding different positive integers, and the assignment of diagnosis results to the corresponding positive integers may be pre-stored in the form of a lookup table. Therefore, during quantification of the diagnosis result of a patient to whom a sample belongs, the lookup table is searched for a positive integer corresponding to the diagnosis result of the patient.

In an example, various different departments may also be assigned corresponding different positive integers, and the assignment of departments to the corresponding positive integers may be pre-stored in the form of a lookup table. Therefore, during quantification of the department to which a sample belongs, the lookup table is searched for a positive integer corresponding to the department to which the sample belongs.

An embodiment of the disclosure further provides a computer system for generating a review model for reviewing test data, including a data obtaining module and a model construction module.

The data obtaining module is configured to obtain test data and clinical information of a plurality of samples. Here, the test data of these samples is unlabeled, i.e., unannotated or unmarked. Moreover, the test data includes a test result for at least one test parameter, and the clinical information at least includes at least one of department and diagnosis result, for example, the clinical information at least includes department and diagnosis result.

The data obtaining module is specifically configured to implement step S110 of the method 100 or one of its embodiments.

The model construction module is configured to, for each test parameter of the at least one test parameter, construct a review model corresponding to said test parameter by using a machine learning algorithm based on the test result corresponding to said test parameter in the test data of each sample of the plurality of samples and the clinical information of said sample.

The model construction module is specifically configured to implement step S120 of the method 100 or one of its embodiments.

As shown in FIG. 12, an embodiment of the disclosure further provides a method 1200 for reviewing test data using a review model constructed based on the method of any one of the aforementioned embodiments, including the following steps:

Step S1210: obtaining a test result for a current test parameter of a current sample and clinical information of the current sample.
Step S1220: determining a current review model from a plurality of review models based on the current test parameter and the clinical information, wherein the plurality of review models are constructed according to the method of any one of the aforementioned embodiments.
Step S1230: performing a review on the test result for the current test parameter based on the current review model.
Step S1240: outputting, based on a result of the review, the test result for the current test parameter or an alarm prompt and/or a manual review prompt.

In other words, for reviewing the result of a test parameter for any sample, a corresponding review model is found based on the test parameter and the input clinical information (including gender, age, department, diagnosis, etc.), to obtain the review result of the test parameter based on the review model. This enables efficient result review.

Optionally, the method 1200 further includes outputting the current review model.

In some embodiments, in step S1240, outputting, based on a result of the review, the test result for the current test parameter or an alarm prompt and/or a manual review prompt, may include:
when the current test parameter includes only a single test parameter, that is, when the current test parameter is a single test parameter, outputting the test result for the single test parameter if the test result for the single test parameter is determined to be qualified based on the current review model, otherwise outputting the alarm prompt and/or the manual review prompt.

In some other embodiments, in step S1240, outputting, based on a result of the review, the test result for the current test parameter or an alarm prompt and/or a manual review prompt, may include:

when the current test parameter includes a plurality of test parameters, outputting test results for the plurality of test parameters if the test result for each of the test parameters is determined to be qualified based on the current review model; or outputting the alarm prompt and/or the manual review prompt if the test result for at least one test parameter of the plurality of test parameters is determined to be unqualified based on the current review model.

In other words, in the case where the current test parameter includes a plurality of test parameters, all the test parameters need to be traversed to find a review model corresponding to each test parameter in order to complete result review for said test parameter. The current sample passes the review only if the results for all the test parameters of the current sample pass the review; conversely, as long as the result for even one of the test parameters is unqualified, the current sample fails the review.

It should be understood here that when the current test parameter includes a plurality of test parameters, there may be only one determined current review model (for example, when the plurality of test parameters are grouped into a same parameter group), or there may be a plurality of determined current review models (for example, when the plurality of test parameters are grouped into at least two parameter groups).

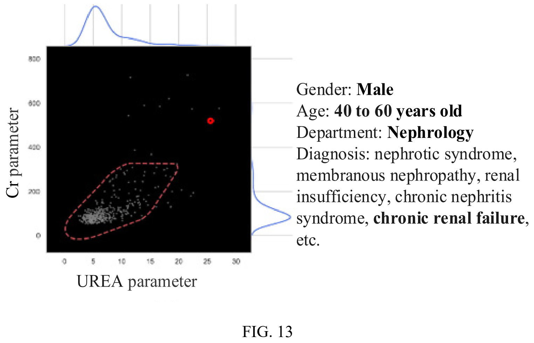

In an example, the aforementioned method 1200 is used to perform a review on test results for test parameters of a patient sample shown in Table 5. The test parameters include UREA and Cr. According to one of the aforementioned embodiments, UREA and Cr are grouped into a same group. A corresponding UREA-Cr parameter-group review model is found based on the clinical information of the patient sample, as shown in FIG. 11. Then, based on the position of the UREA test result and the Cr test result for the patient sample in the review model shown in FIG. 11, as indicated by the red dot in FIG. 13, it is determined that the UREA parameter and the Cr parameter of the patient sample fail the review (the red dot is outside the contour line shown by the red dash line).

Table 5. Clinical Information and Test Data of a Certain Patient Sample

| Gender | Age | Department(s) | Diagnosis Result(s) | UREA | Cr |
|---|---|---|---|---|---|
| Male | 50 years old | Nephrology | Chronic Renal Failure | 25.47 | 531.1 |

An embodiment of the disclosure further provides a computer system for reviewing test data, including a data obtaining module, a model determination module, a review module, and an output module.

The data obtaining module is configured to obtain a test result for a current test parameter of a current sample and clinical information corresponding to the current sample. In particular, the data obtaining module is configured to implement step S1210 of the method 1200.

The model determination module is configured to determine a current review model from a plurality of review models based on the current test parameter and the clinical information, wherein the plurality of review models are constructed according to the method of any one of the aforementioned embodiments. For example, the review models constructed according to the method of any one of the aforementioned embodiments are stored in a memory for retrieval from the memory when needed. In particular, the data obtaining module is configured to implement step S1220 of the method 1200.

The review module is configured to review the test result for the current test parameter based on the current review model. In particular, the data obtaining module is configured to implement step S1230 of the method 1200.

The output module is configured to output, based on a result of the review, the test result for the current test parameter or an alarm prompt and/or a manual review prompt. In particular, the data obtaining module is configured to implement step S1240 of the method 1200.

In the embodiments of the disclosure, the various modules of the computer system may be implemented in software and/or hardware.

In some embodiments, the computer system may be implemented in the form of middleware.

In some embodiments, the computer system may be integrated into a laboratory (clinical laboratory department) information system (LIS) or a hospital information system (HIS) in the form of software.

In some other embodiments, the computer system may be integrated into the LIS system or HIS system in the form of hardware, for example, a processor or a computer-readable storage medium, and the respective modules thereof may be implemented in the hardware in the form of code or software.

In still some other embodiments, the computer system may also be a stand-alone hardware device.

An embodiment of the disclosure further provides an apparatus for generating a review model for reviewing test data, including: a non-transitory computer-readable storage medium storing computer-readable instructions; and one or more processors configured to execute the computer-readable instructions to implement the method 100 of any one of the aforementioned embodiments.

An embodiment of the disclosure further provides an apparatus for reviewing test data, including: a non-transitory computer-readable storage medium storing computer-readable instructions; and one or more processors configured to execute the computer-readable instructions to implement the method 1200 of any one of the aforementioned embodiments.

An embodiment of the disclosure further provides a computer-readable storage medium having a computer program stored thereon, wherein the computer program, when executed by a processor, implements the method 100 or the method 1200 according to any one of the aforementioned embodiments.

Furthermore, it should be understood for those skilled in the art that the modules or steps of the embodiments of the disclosure may be implemented using a general-purpose computing apparatus. The general-purpose computing apparatus typically includes a processor and a memory. The memory is configured to store instructions, wherein the instructions, when executed by the processor, cause the computing apparatus to perform the steps or program modules of the embodiments of the disclosure.

In the embodiments of the disclosure, the processor may be a central processing unit (CPU), or the processor may be another general-purpose processor, a digital signal processor (DSP), an application-specific integrated circuit (ASIC), a field-programmable gate array (FPGA), or another programmable logic device, a discrete gate or transistor logic device, a discrete hardware component, or the like. The general-purpose processor may be a microprocessor, or the processor may be any conventional processor, etc.

In the embodiments of the disclosure, the memory is configured to store computer programs or instructions, and the memory may be: a volatile memory, such as a random access memory (RAM), or a non-volatile memory, such as a read-only memory (ROM), a flash memory, a hard disk drive (HDD), or a solid-state drive (SSD), or a combination of the above types of memories.

A person skilled in the art understands that the embodiments of the disclosure may be provided as a method, a system, or a computer program product. Therefore, the embodiments of the disclosure may be implemented in the form of a hardware embodiment, a software embodiment, or a combination thereof. Moreover, the embodiments of the disclosure may be implemented in the form of a computer program product that is implemented on one or more computer-usable storage media (including a disk memory, an optical memory, and the like) that include computer-usable program code.

The embodiments of the disclosure are described with reference to flowcharts and/or block diagrams of the methods, devices (systems), and computer program products according to the embodiments of the disclosure. It should be understood that each procedure and/or block in the flowcharts and/or block diagrams, and combinations of the procedures and/or blocks in the flowcharts and/or block diagrams may be implemented by computer program operations. These computer program operations may be provided to a processor of a general-purpose computer, a special-purpose computer, an embedded processor or other programmable data processing devices to create a machine, such that the operations executed by the processor of the computer or other programmable data processing devices create a device for implementing functions specified in one or more procedures in the flowcharts and/or one or more blocks in the block diagrams.

These computer program operations may also be stored in a computer-readable memory that may direct a computer or other programmable data processing device to operate in a specific manner, such that the operations stored in the computer-readable memory create a manufacture article including an operation device, and the operation device implements the functions specified in one or more procedures in the flowcharts and/or one or more blocks in the block diagrams.

These computer program operations may also be loaded onto a computer or other programmable data processing devices to enable a series of operation steps to be executed on the computer or other programmable devices to perform computer-implemented processing, such that the operations executed on the computer or other programmable devices provide steps for implementing the functions specified in one or more procedures in the flowcharts and/or one or more blocks in the block diagrams.

The features or combinations thereof mentioned above in the description, accompanying drawings, and claims can be combined with each other arbitrarily or used separately as long as they are meaningful within the scope of the disclosure and do not contradict each other. The advantages and features described with respect to the method provided in the embodiments of the disclosure are correspondingly applicable to the apparatuses and computer-readable storage medium provided in the embodiments of the disclosure, and vice versa.

The foregoing description merely relates to the preferred embodiments of the disclosure, and is not intended to limit the scope of patent of the disclosure. All equivalent variations made by using the content of the specification and the accompanying drawings of the disclosure from the concept of the disclosure, or the direct/indirect applications of the contents in other related technical fields all fall within the scope of patent protection of the disclosure.

**EXAMPLES**

1. A method for generating a review model for reviewing test data, characterized in that the method comprises:

obtaining test data and clinical information of a plurality of samples, wherein the test data is unlabeled, the test data comprises a test result for at least one test parameter, and the clinical information at least comprises at least one of department and diagnosis result; and

for each test parameter of the at least one test parameter, constructing a review model corresponding to said test parameter by using a machine learning algorithm based on the test result corresponding to said test parameter in the test data of each sample of the plurality of samples and the clinical information of said sample.

2. The method of example 1, characterized in that the clinical information further comprises gender and age, and preferably, the clinical information comprises department, diagnosis result, gender, and age.

3. The method of example 1 or 2, characterized in that constructing a review model corresponding to said test parameter by using a machine learning algorithm based on the test result corresponding to said test parameter in the test data of each sample of the plurality of samples and the clinical information of said sample, comprises:

performing clustering on the clinical information of the plurality of samples; and

constructing the review model corresponding to said test parameter based on the test result corresponding to said test parameter in the test data of each sample of the plurality of samples and the clustered clinical information of said sample, preferably, based on a distribution of the test result corresponding to said test parameter in the test data of each sample of the plurality of samples across the clustered clinical information.

4. The method of example 3, characterized in that the clinical information at least comprises department, and performing clustering on the clinical information of the plurality of samples comprises performing clustering on the departments of the plurality of samples; and

preferably, the clinical information at least comprises department and diagnosis result, and performing clustering on the clinical information of the plurality of samples comprises respectively performing clustering on the departments of the plurality of samples and the diagnosis results of the plurality of samples.

5. The method of example 3 or 4, characterized in that performing clustering on the clinical information of the plurality of samples, comprises:

performing clustering on the clinical information of the plurality of samples based on a preset clustering rule, and optionally, performing clustering on the clinical information of the plurality of samples based on a manually set clustering rule; or

performing clustering, and preferably performing K-means clustering, on the clinical information of the plurality of samples based on the test result corresponding to said test parameter in the test data of each sample of the plurality of samples.

6. The method of any one of examples 3 to 5, characterized in that the at least one test parameter comprises a plurality of test parameters, and prior to performing clustering on the clinical information of the plurality of samples, the method further comprises: grouping the plurality of test parameters to obtain a plurality of parameter groups, each parameter group consisting of a single test parameter of the plurality of test parameters or consisting of multiple test parameters of the plurality of test parameters; and

constructing the review model corresponding to said test parameter based on the test result corresponding to said test parameter in the test data of each sample of the plurality of samples and the clustered clinical information of said sample, comprises: constructing a review model corresponding to the parameter group containing said test parameter based on a distribution of the test result corresponding to each test parameter in the parameter group containing said test parameter in the test data of each sample of the plurality of samples across the clustered clinical information, as the review model corresponding to said test parameter.

7. The method of example 6, characterized in that grouping the plurality of test parameters to obtain a plurality of parameter groups comprises:

grouping the plurality of test parameters based on a preset grouping rule, and optionally, grouping the plurality of test parameters based on a manually set grouping rule; or

grouping the plurality of test parameters based on a correlation between the test results for every two test parameters of the plurality of test parameters.

8. The method of example 6 or 7, characterized in that for each test parameter of the at least one test parameter, constructing a review model corresponding to said test parameter by using a machine learning algorithm based on the test result corresponding to said test parameter in the test data of each sample of the plurality of samples and the clinical information of said sample, comprises:

performing clustering on the clinical information of the plurality of samples based on the test result corresponding to each test parameter in the parameter group containing said test parameter in the test data of each sample of the plurality of samples;

classifying the plurality of samples, and preferably classifying the plurality of samples based on the clustered clinical information, to obtain a plurality of sample groups; and

determining a review model corresponding to each sample group based on a distribution of the test result corresponding to each test parameter in the parameter group containing said test parameter in test data of each sample in said sample group across the clustered clinical information, as the review model corresponding to said test parameter.

9. The method of example 8, characterized in that the parameter group containing said test parameter comprises a parameter group consisting of a single test parameter; and

determining a review model corresponding to each sample group based on a distribution of the test result corresponding to each test parameter in the parameter group containing said test parameter in test data of each sample in said sample group across the clustered clinical information, comprises:

determining a review threshold corresponding to each sample group based on a one-dimensional distribution of the test result corresponding to the single test parameter in the parameter group consisting of the single test parameter in the test data of each sample in said sample group across the clustered clinical information, to determine the review model corresponding to said sample group.

10. The method of example 8, characterized in that the parameter group containing the test parameter comprises a parameter group consisting of multiple test parameters; and

determining a review model corresponding to each sample group based on a distribution of the test result corresponding to each test parameter in the parameter group containing said test parameter in test data of each sample in said sample group across the clustered clinical information, comprises:

determining a review threshold corresponding to each sample group based on a multi-dimensional distribution of the test results corresponding to the multiple test parameters in the parameter group consisting of the multiple test parameters in the test data of each sample in the sample group across the clustered clinical information, to determine the review model corresponding to said sample group; or

determining a review threshold corresponding to each sample group based on a one-dimensional distribution of the test results corresponding to the multiple test parameters in the parameter group consisting of the multiple test parameters in the test data of each sample in the sample group across the clustered clinical information, to determine the review model corresponding to said sample group.

11. The method of any one of examples 1 to 10, characterized in that prior to constructing the review model, data preprocessing is performed on the test data and the clinical information of each sample of the plurality of samples, the data preprocessing comprising at least one of data cleaning, data deduplication, data imputation, and quantification of the clinical information, so as to construct the review model based on the preprocessed test data and the preprocessed clinical information.

12. The method of any one of examples 1 to 11, characterized in that the test data being unlabeled indicates that the test data is not annotated with at least one of whether retesting is performed, whether an abnormality is present, and whether review approval is granted.

13. A method for reviewing test data, characterized in that the method comprises:

obtaining a test result for a current test parameter of a current sample and clinical information of the current sample;

determining a current review model from a plurality of review models based on the current test parameter and the clinical information, wherein the plurality of review models are constructed according to the method of any one of examples 1 to 11;

performing a review on the test result for the current test parameter based on the current review model; and

outputting, based on a result of the review, the test result for the current test parameter or an alarm prompt and/or a manual review prompt.

14. The method of example 13, characterized in that outputting, based on a result of the review, the test result for the current test parameter or an alarm prompt and/or a manual review prompt, comprises:

when the current test parameter is a single test parameter, outputting the test result for the single test parameter if the test result for the single test parameter is determined to be qualified based on the current review model, otherwise outputting the alarm prompt and/or the manual review prompt; or
when the current test parameter comprises a plurality of test parameters, outputting the test results for the plurality of test parameters if the test result for each of the test parameters is determined to be qualified based on the current review model; or outputting the alarm prompt and/or the manual review prompt if the test result for at least one test parameter of the plurality of test parameters is determined to be unqualified based on the current review model.

15. The method of example 13 or 14, characterized in that the method further comprises outputting the current review model.

16. An apparatus for generating a review model for reviewing test data, characterized in that the apparatus comprises:

a non-transitory computer-readable storage medium storing computer-readable instructions; and
one or more processors configured to execute the computer-readable instructions to implement the method of any one of examples 1 to 12.

17. An apparatus for reviewing test data, characterized in that the apparatus comprises:

a non-transitory computer-readable storage medium storing computer-readable instructions; and
one or more processors configured to execute the computer-readable instructions to implement the method of any one of examples 13 to 15.

18. A computer-readable storage medium having a computer program stored thereon, wherein the computer program, when executed by a processor, implements the method of any one of examples 1 to 15.

**Claims**

1. A method for generating a review model for reviewing test data, **characterized in that** the method comprises:

obtaining test data and clinical information of a plurality of samples, wherein the test data is unlabeled, the test data comprises a test result for at least one test parameter, and the clinical information at least comprises at least one of department and diagnosis result; and
for each test parameter of the at least one test parameter, constructing a review model corresponding to said test parameter by using a machine learning algorithm based on the test result corresponding to said test parameter in the test data of each sample of the plurality of samples and the clinical information of said sample.

2. The method of claim 1, **characterized in that** the clinical information further comprises gender and age, and preferably, the clinical information comprises department, diagnosis result, gender, and age.

3. The method of claim 1 or 2, **characterized in that** constructing a review model corresponding to said test parameter by using a machine learning algorithm based on the test result corresponding to said test parameter in the test data of each sample of the plurality of samples and the clinical information of said sample, comprises:

performing clustering on the clinical information of the plurality of samples; and
constructing the review model corresponding to said test parameter based on the test result corresponding to said test parameter in the test data of each sample of the plurality of samples and the clustered clinical information of said sample, preferably, based on a distribution of the test result corresponding to said test parameter in the test data of each sample of the plurality of samples across the clustered clinical information.
preferably, the clinical information at least comprises department, and performing clustering on the clinical information of the plurality of samples comprises performing clustering on the departments of the plurality of samples; and

preferably, the clinical information at least comprises department and diagnosis result, and performing clustering on the clinical information of the plurality of samples comprises respectively performing clustering on the departments of the plurality of samples and the diagnosis results of the plurality of samples.

4. The method of claim 3, **characterized in that** performing clustering on the clinical information of the plurality of samples, comprises:

performing clustering on the clinical information of the plurality of samples based on a preset clustering rule, and optionally, performing clustering on the clinical information of the plurality of samples based on a manually set clustering rule; or

performing clustering, and preferably performing K-means clustering, on the clinical information of the plurality of samples based on the test result corresponding to said test parameter in the test data of each sample of the plurality of samples.

5. The method of claim 3 or 4, **characterized in that** the at least one test parameter comprises a plurality of test parameters, and prior to performing clustering on the clinical information of the plurality of samples, the method further comprises: grouping the plurality of test parameters to obtain a plurality of parameter groups, each parameter group consisting of a single test parameter of the plurality of test parameters or consisting of multiple test parameters of the plurality of test parameters; and

constructing the review model corresponding to said test parameter based on the test result corresponding to said test parameter in the test data of each sample of the plurality of samples and the clustered clinical information of said sample, comprises: constructing a review model corresponding to the parameter group containing said test parameter based on a distribution of the test result corresponding to each test parameter in the parameter group containing said test parameter in the test data of each sample of the plurality of samples across the clustered clinical information, as the review model corresponding to said test parameter.

preferably, grouping the plurality of test parameters to obtain a plurality of parameter groups comprises:

grouping the plurality of test parameters based on a preset grouping rule, and optionally, grouping the plurality of test parameters based on a manually set grouping rule; or

grouping the plurality of test parameters based on a correlation between the test results for every two test parameters of the plurality of test parameters.

6. The method of claim 5, **characterized in that** for each test parameter of the at least one test parameter, constructing a review model corresponding to said test parameter by using a machine learning algorithm based on the test result corresponding to said test parameter in the test data of each sample of the plurality of samples and the clinical information of said sample, comprises:

performing clustering on the clinical information of the plurality of samples based on the test result corresponding to each test parameter in the parameter group containing said test parameter in the test data of each sample of the plurality of samples;

classifying the plurality of samples, and preferably classifying the plurality of samples based on the clustered clinical information, to obtain a plurality of sample groups; and

determining a review model corresponding to each sample group based on a distribution of the test result corresponding to each test parameter in the parameter group containing said test parameter in test data of each sample in said sample group across the clustered clinical information, as the review model corresponding to said test parameter.

7. The method of claim 6, **characterized in that** the parameter group containing said test parameter comprises a parameter group consisting of a single test parameter; and

determining a review model corresponding to each sample group based on a distribution of the test result corresponding to each test parameter in the parameter group containing said test parameter in test data of each sample in said sample group across the clustered clinical information, comprises:

determining a review threshold corresponding to each sample group based on a one-dimensional distribution of the test result corresponding to the single test parameter in the parameter group consisting of the single test parameter in the test data of each sample in said sample group across the clustered clinical information, to determine the review model corresponding to said sample group.

8. The method of claim 7, **characterized in that** the parameter group containing the test parameter comprises a parameter group consisting of multiple test parameters; and
determining a review model corresponding to each sample group based on a distribution of the test result corresponding to each test parameter in the parameter group containing said test parameter in test data of each sample in said sample group across the clustered clinical information, comprises:

determining a review threshold corresponding to each sample group based on a multi-dimensional distribution of the test results corresponding to the multiple test parameters in the parameter group consisting of the multiple test parameters in the test data of each sample in the sample group across the clustered clinical information, to determine the review model corresponding to said sample group; or
determining a review threshold corresponding to each sample group based on a one-dimensional distribution of the test results corresponding to the multiple test parameters in the parameter group consisting of the multiple test parameters in the test data of each sample in the sample group across the clustered clinical information, to determine the review model corresponding to said sample group.

9. The method of any one of claims 1 to 8, **characterized in that** prior to constructing the review model, data preprocessing is performed on the test data and the clinical information of each sample of the plurality of samples, the data preprocessing comprising at least one of data cleaning, data deduplication, data imputation, and quantification of the clinical information, so as to construct the review model based on the preprocessed test data and the preprocessed clinical information; and/or
**characterized in that** the test data being unlabeled indicates that the test data is not annotated with at least one of whether retesting is performed, whether an abnormality is present, and whether review approval is granted.

10. A method for reviewing test data, **characterized in that** the method comprises:

obtaining a test result for a current test parameter of a current sample and clinical information of the current sample;
determining a current review model from a plurality of review models based on the current test parameter and the clinical information, wherein the plurality of review models are constructed according to the method of any one of claims 1 to 9;
performing a review on the test result for the current test parameter based on the current review model; and
outputting, based on a result of the review, the test result for the current test parameter or an alarm prompt and/or a manual review prompt.

11. The method of claim 10, **characterized in that** outputting, based on a result of the review, the test result for the current test parameter or an alarm prompt and/or a manual review prompt, comprises:

when the current test parameter is a single test parameter, outputting the test result for the single test parameter if the test result for the single test parameter is determined to be qualified based on the current review model, otherwise outputting the alarm prompt and/or the manual review prompt; or
when the current test parameter comprises a plurality of test parameters, outputting the test results for the plurality of test parameters if the test result for each of the test parameters is determined to be qualified based on the current review model; or outputting the alarm prompt and/or the manual review prompt if the test result for at least one test parameter of the plurality of test parameters is determined to be unqualified based on the current review model.

12. The method of claim 10 or 11, **characterized in that** the method further comprises outputting the current review model.

13. An apparatus for generating a review model for reviewing test data, **characterized in that** the apparatus comprises:

a non-transitory computer-readable storage medium storing computer-readable instructions; and
one or more processors configured to execute the computer-readable instructions to implement the method of any one of claims 1 to 9.

14. An apparatus for reviewing test data, **characterized in that** the apparatus comprises:

a non-transitory computer-readable storage medium storing computer-readable instructions; and
one or more processors configured to execute the computer-readable instructions to implement the method of any one of claims 10 to 12.

15. A computer-readable storage medium having a computer program stored thereon, wherein the computer program, when executed by a processor, implements the method of any one of claims 1 to 12.

100

| S110 | obtaining test data and clinical information of a plurality of samples |
|---|---|

| S120 | for each test parameter, constructing a review model corresponding to said test parameter by using a machine learning algorithm based on the test result corresponding to said test parameter in test data of each sample and clinical information of said sample |
|---|---|

FIG. 1

FIG. 2

FIG. 3

UA_CO2 parameter distribution

FIG. 4

UREA_Cr parameter distribution

FIG. 5

UREA_CYSC parameter distribution

FIG. 6

Cr_CYSC parameter distribution

FIG. 7

CO2 parameter distribution curve

Gender: Female
Age: 40 to 60 years old
Department: orthopedics and joint surgery, spine surgery, comprehensive ward, gastrointestinal surgery, hematologic, etc.
Diagnosis: clavicle fracture, cervical spondylosis, chemotherapy for malignant lymphoma, diabetic ketoacidosis, etc.

FIG. 8

CO2 parameter distribution curve

Gender: Male
Age: 40 to 60 years old
Department: cardiovascular medicine, infectious diseases
department, radiation therapy department, geriatrics medicine,
general medicine, etc.
Diagnosis: chest tightness, arrhythmia, chronic hepatitis B,
hypertension, hyperlipidemia, etc.

FIG. 9

Gender: Female
Age: 60 to 80 years old
Department: sports medicine, orthopedics and joint surgery,
respiratory medicine, special clinic, neurology, etc.
Diagnosis: central nervous system demyelinating disease,
pulmonary infection, limb numbness, jaw malformation, etc.

FIG. 10

Gender: Male
Age: 40 to 60 years old
Department: Nephrology
Diagnosis: nephrotic syndrome, membranous nephropathy, renal insufficiency, chronic nephritis syndrome, chronic renal failure, etc.

FIG. 11

1200

S1210 — obtaining a test result for a current test parameter of a current sample and clinical information of the current sample

S1220 — determining a current review model from a plurality of review model based on the current test parameter and the clinical information

S1230 — performing a review on the test result for the current test parameter based on the current review model

S1240 — outputting, based on a result of the review, the test result for the current test parameter or an alarm prompt and/or a manual review prompt

FIG. 12

Gender: **Male**
Age: **40 to 60 years old**
Department: **Nephrology**
Diagnosis: nephrotic syndrome, membranous nephropathy, renal insufficiency, chronic nephritis syndrome, **chronic renal failure**, etc.

FIG. 13

Europäisches
Patentamt
European
Patent Office
Office européen
des brevets

## EUROPEAN SEARCH REPORT

**Application Number**

EP 25 22 4732

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZHU JING ET AL: "Combined strategy of knowledge-based rule selection and historical data percentile-based range determination to improve an autoverification system for clinical chemistry test results", JOURNAL OF CLINICAL LABORATORY, vol. 36, no. 2, 10 January 2022 (2022-01-10), XP093397952, US ISSN: 0887-8013, DOI: 10.1002/jcla.24233 * the whole document * | 1-15 | INV. G16H10/20 G16H10/40 |
| A | WANG HONGCHUN ET AL: "Using machine learning to develop an autoverification system in a clinical biochemistry laboratory", CLINICAL CHEMISTRY AND LABORATORY MEDICINE (CCLM), vol. 59, no. 5, 26 November 2020 (2020-11-26), pages 883-891, XP093397951, ISSN: 1434-6621, DOI: 10.1515/cclm-2020-0716 Retrieved from the Internet: URL:https://www.researchgate.net/profile/C hengxi-Sun/publication/347269681_Using_mac hine_learning_to_develop_an_autoverificati on_system_in_a_clinical_biochemistry_labor atory/links/64db2d8e78e40b48bd4b5028/Using -machine-learning-to-develop-an-autoverifi cation-system-in-a-clinical-biochemistry-l aboratory.> * the whole document * | 1-15 | |

**TECHNICAL FIELDS SEARCHED (IPC)**

G16H

The present search report has been drawn up for all claims

| Place of search | Date of completion of the search | Examiner |
|---|---|---|
| Munich | 20 May 2026 | Rinelli, Pietro |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P4C01)